# EUROPÄISCHE PATENTSCHRIFT

(11) **EP 2 364 170 B1**
(45) Veröffentlichungstag und Bekanntmachung des Hinweises auf die Patenterteilung: **20.03.2013**
(21) Anmeldenummer: 09760481.3
(22) Anmeldetag: 06.11.2009
(51) Int. Cl.: A61L 17/04, A61L 17/14

(54) **CHIRURGISCHER FADEN, INSBESONDERE ZUR VERMEIDUNG VON STICHKANALBLUTUNGEN, UND VERFAHREN ZU SEINER HERSTELLUNG**
SURGICAL THREAD, IN PARTICULAR FOR PREVENTING PUNCTURE CHANNEL BLEEDING, AND A METHOD FOR PRODUCING SAME
FIL CHIRURGICAL, EN PARTICULIER DESTINÉ À ÉVITER LES SAIGNEMENTS DU TRAJET DE L'AIGUILLE, ET SON PROCÉDÉ DE FABRICATION

(30) Priorität: 06.11.2008 DE 102008057214
(43) Veröffentlichungstag der Anmeldung: 14.09.2011
(73) Patentinhaber: ITV Denkendorf Produktservice GmbH, 73770 Denkendorf (DE)
(72) Erfinder: OBERHOFFNER, Sven, 71384 Weinstadt (DE); PLANCK, Heinrich, 72622 Nürtingen (DE); MÜLLER, Erhard, 70565 Stuttgart (DE)
(74) Vertreter: Patentanwälte Ruff, Wilhelm, Beier, Dauster & Partner
(86) Internationale Anmeldenummer: PCT/EP2009/007947
(87) Internationale Veröffentlichungsnummer: WO 2010/052004

(56) Entgegenhaltungen:
- WO-A2-2006/138300
- US-B1- 6 183 499

## Beschreibung

Die vorliegende Erfindung betrifft einen chirurgischen Faden mit einem polymeren Kern-Mantel-Aufbau, welcher sich insbesondere zur Vermeidung von Stichkanalblutungen eignet, ein entsprechendes chirurgisches Kit, Herstellungsverfahren für den chirurgischen Faden sowie die Verwendung des Fadens als chirurgisches Nahtmaterial.

Zum Verschluss von Wunden kommen in der modernen Versorgungschirurgie standardmäßig fadenförmige Nahtmaterialien in Kombination mit chirurgischen Nadeln zum Einsatz. Beim Einstechen der Nadel in ein biologisches Gewebe entsteht ein sogenannter Stichkanal, durch den das Nahtmaterial nachgezogen wird. Da in den meisten Fällen der Nadeldurchmesser größer ist als der Durchmesser des Nahtmaterials, wird der von der Nadel geformte Stichkanal durch das Nahtmaterial nicht vollständig ausgefüllt. Dies kann zu sogenannten Stichkanalblutungen führen, was insbesondere bei kardiovaskulären Nähten zu Komplikationen führen kann. Darüber hinaus bietet ein durch das Nahtmaterial nicht vollständig ausgefüllter Stichkanal Kolonisierungsmöglichkeiten für Infektionserreger, wodurch sich das Risiko von post-operativen Infektionen stark erhöht.

Ein Nahtmaterial auf der Basis eines resorbierbaren, amphiphilen Blockcopolymers mit einem Mittelblock aus Polyethylenglykol und terminalen Blöcken aus Glykolid, Lactid, ε-Caprolacton, p-Dioxanon, Trimethylencarbonat und/oder Morpholindion ist aus der US 2007/0275034 A1 bekannt. Nachteilig hierbei ist jedoch das an sich schwache Quellverhalten des Nahtmaterials in Körperflüssigkeiten, was seine Einsatzmöglichkeiten hinsichtlich der Vermeidung von Stichkanalblutungen stark schmälert. Abgesehen davon, wird das mögliche Auftreten von Stichkanalblutungen in der US-Offenlegungsschrift überhaupt nicht problematisiert.

Aus der WO 2006/138300 A2 ist unter anderem ein in physiologischen Medien quellbares Nahtmaterial mit einem schichtförmigen Aufbau bekannt. Die quellbaren Eigenschaften des Nahtmaterials beruhen dabei auf einer hydrophilen Außenschicht. Diese wird durch eine Propf-Polymerisation hergestellt und besitzt ein polymeres Netzwerk mit ionischen Gruppen. Aufgrund des osmotischen Drucks der umgebenden Körperflüssigkeit verursacht das ionische Gruppen enthaltende Netzwerk, das im Wesentlichen einen kovalent mit dem Fadenkern verbundenen Superabsorber darstellt, eine sehr schnelle Quellung des Nahtmaterials im Körper. Dies kann dazu führen, dass das Nahtmaterial schon beim Durchzug durch ein Gewebe einen bedeutend größeren Durchmesser annimmt als eine am Nahtmaterialende befestigte Nadel. Hierdurch wird eine hohe Durchzugskraft erforderlich, die zu einer verstärkten Traumatisierung des Gewebes führen kann. Außerdem wird eine Nachplatzierbarkeit des Knotens kaum zu erzielen sein. Ferner stehen die ionischen Gruppen des kovalent gebundenen Superabsorbers in direktem Kontakt zu den Körperflüssigkeiten, insbesondere zu Blut, und können aufgrund von Wechselwirkungen beispielsweise die Blutgerinnung negativ beeinflussen oder unerwünschte lonenaustauschvorgänge hervorrufen und somit wichtige im Körper ablaufende Prozesse beeinträchtigen. Außerdem ist die Pfropf-Polymerisation ein Herstellungsverfahren, das allgemein aufwendig und kostenintensiv ist. Ein weiterer Nachteil besteht darin, dass sich durch eine Pfropf-Polymerisation in der Regel keine einheitliche Schichtdicke erzielen lässt, was zu ungleichmäßigen Ausdehnungstendenzen des Nahtmaterials in physiologischen Medien führen kann. Dies wiederum schränkt die Verwendungsmöglichkeiten hinsichtlich der Verhinderung von Stichkanalblutungen ein.

Der vorliegenden Erfindung liegt daher die Aufgabe zugrunde, einen chirurgischen Faden bereitzustellen, der Stichkanalblutungen verhindert, ohne dass hierbei aus dem Stand der Technik bekannte Nachteile auftreten. Gleichzeitig soll der Faden eine erhöhte Knotensicherheit sowie einen verbesserten Knotenhalt bewirken. Des Weiteren liegt der vorliegenden Erfindung auch die Aufgabe zugrunde, Herstellungsverfahren für den chirurgischen Faden bereitzustellen, welche im Vergleich zu herkömmlichen Verfahren möglichst einfach und kostengünstig durchführbar sind.

Diese Aufgabe wird gelöst durch einen chirurgischen Faden mit den Merkmalen des unabhängigen Anspruchs 1. Bevorzugte Ausführungsformen des erfindungsgemäßen Fadens sind Gegenstand der abhängigen Ansprüche 2 bis 12. Die vorliegende Erfindung betrifft auch ein chirurgisches Kit mit den Merkmalen gemäß unabhängigem Anspruch 13. Ein weiterer Aspekt der vorliegenden Erfindung betrifft Herstellungsverfahren für den chirurgischen Faden gemäß den unabhängigen Ansprüchen 14 bis 16. Der Wortlaut sämtlicher Ansprüche wird hiermit durch Bezugnahme zum Inhalt dieser Beschreibung gemacht.

Bei dem erfindungsgemäßen Faden handelt es sich um einen chirurgischen Faden, vorzugsweise um ein chirurgisches Nahtmaterial, insbesondere zur Vermeidung von Stichkanalblutungen, mit einem polymeren Fadenkern und einer den polymeren Fadenkern umgebenden polymeren Ummantelung (Kern-Mantel-Aufbau), wobei die polymere Ummantelung in Körperflüssigkeiten quellbar ist bzw. quellbar ausgebildet ist.

Mit anderen Worten wird durch die vorliegende Erfindung ein chirurgischer Faden, vorzugsweise in Form eines chirurgischen
Nahtmaterials, mit einem polymeren Kern-Mantel-Aufbau bereitgestellt, dessen Mantel in Körperflüssigkeiten quellbar ist bzw. quellbar ausgebildet ist. Durch die quellbare Ausgestaltung der Ummantelung dehnt sich der chirurgische Faden bei Kontakt mit Körperflüssigkeiten, insbesondere Blut, vorzugsweise kontrolliert aus, wodurch sich insgesamt der Durchmesser des erfindungsgemäßen Fadens vergrößert. Wird der erfindungsgemäße Faden somit durch einen durch eine chirurgische Nadel gebildeten Stichkanal hindurchgezogen und verknotet, so bewirken die hierbei in der Regel auftretenden Stichkanalblutungen eine Quellung der Ummantelung. Die hieraus resultierende Ausdehnung bzw. Durchmesservergrößerung des Fadens führt mit besonderem Vorteil zu einem vollständigen und dichten Verschluss des Stichkanals. Dadurch können keine Infektionserreger in den Stichkanal eindringen, wodurch das Risiko von post-operativen Infektionen deutlich reduziert wird. Erfindungsgemäß kann es dabei durchaus vorgesehen sein, dass sich der Faden infolge der Quellbarkeit der Ummantelung stärker ausdehnt als es dem Durchmesser des Stichkanals entspricht. Dadurch drückt die polymere Ummantelung gegen die Stichkanalwand, wodurch sich eine besonders gute Abdichtung des Stichkanals ergibt. Ein weiterer Vorteil des erfindungsgemäßen Fadens besteht darin, dass in die quellbare Ummantelung eingedrungenes Blut in der Regel gerinnt, wodurch der Stichkanal trocken gehalten und beispielsweise die Entstehung und gegebenenfalls Ansammlung von Wundwasser (Exsudat) im Stichkanal vermieden wird. Ein weiterer Vorteil, der sich aus den besonderen Quelleigenschaften der Ummantelung ergibt, besteht darin, dass die Quellung nicht zu schnell, also im Wesentlichen nicht vor Setzung des Knotens, erfolgt. Neben einer Verbesserung der Knotensicherheit und des Knotenhalts wird dadurch auch die Nachplatzierbarkeit beim Knoten nicht negativ beeinflusst.

Unter Körperflüssigkeiten im Sinne der vorliegenden Erfindung sollen grundsätzlich alle im menschlichen und/oder tierischen Körper vorkommenden Flüssigkeiten und darüber hinaus auch alle an sich körperverträglichen Flüssigkeiten verstanden werden. Entsprechend kann es sich bei den Körperflüssigkeiten um Wasser, Blut, Lymphflüssigkeiten, Eiterflüssigkeiten, Exsudate, Urin oder physiologische Pufferlösungen handeln. Bevorzugt handelt es sich bei den Körperflüssigkeiten im Sinne der vorliegenden Erfindung jedoch um menschliches und/oder tierisches Blut.

Erfindungsgemäß kann es grundsätzlich vorgesehen sein, dass die polymere Ummantelung den polymeren Fadenkern nur teilweise umgibt. In der Regel ist der polymere Fadenkern jedoch vollständig, d.h. vollflächig, von der polymeren Ummantelung umgeben.

Der polymere Fadenkern und die polymere Ummantelung berühren sich entlang einer gemeinsamen Grenzfläche (Fläche zwischen polymerem Fadenkern und polymerer Ummantelung), ohne dass der polymere Fadenkern und die polymere Ummantelung kovalent miteinander verbunden sind. Mit anderen Worten ist die Verbindung zwischen polymerem Fadenkern und polymerer Ummantelung frei von kovalenten Bindungen. Eine Verbindung zwischen polymerem Fadenkern und polymerer Ummantelung kann beispielsweise auf reinen Adhäsionskräften beruhen. Erfindungsgemäß kann es insbesondere vorgesehen sein, dass der polymere Fadenkern und die polymere Ummantelung entlang einer gemeinsamen Grenzfläche miteinander verklebt sind. Besonders bevorzugt liegt der chirurgische Faden als Extrusionsfaden, insbesondere als Coextrusionsfaden oder Ummantelungsextrusionsfaden, vor.

In einer weiteren bevorzugten Ausführungsform liegt der Faden als Bikomponentenfaden vor. Unter einem Bikomponentenfaden im Sinne der vorliegenden Erfindung soll dabei ein Faden mit einem polymeren Fadenkern und eine den polymeren Fadenkern umgebende polymere

Ummantelung verstanden werden, wobei Fadenkern und Ummantelung in der Regel jeweils aus einem unterschiedlichen Polymermaterial gebildet sind.

Die polymere Ummantelung besitzt vorzugsweise ein Aufnahmevermögen für Körperflüssigkeiten, dass dem 3- bis 80-fachen, insbesondere 5- bis 40-fachen, ihres trockenen Eigengewichtes entspricht.

In einer bevorzugten Ausführungsform weist die polymere Ummantelung in Körperflüssigkeiten quellbare Additive, vorzugsweise Superabsorbentien bzw. Superabsorber, auf. Die Verwendung von Superabsorbentien bewirkt in besonders vorteilhafter Weise eine zusätzliche Verbesserung der Quellbarkeit der polymeren Ummantelung und damit auch eine stärkere Ausdehnung des Fadendurchmessers. Erfindungsgemäß können die Superabsorbentien eine Partikelgröße zwischen 1 und 100 µm, insbesondere 5 und 50 µm, aufweisen. Zweckmäßigerweise besitzen die erfindungsgemäß in Frage kommenden Superabsorbentien bioverträgliche Eigenschaften. Geeignete Superabsorbentien können ein Aufnahmevermögen für Flüssigkeiten aufweisen, das dem mehr als 100-fachen ihres trockenen Eigengewichtes entspricht. Die polymere Ummantelung weist vorzugsweise einen Anteil an in Körperflüssigkeiten quellbaren Additiven, insbesondere Superabsorbentien, zwischen 2 und 20 Gew.-%, insbesondere 3 und 8 Gew.-%, bezogen auf das Gesamtgewicht der polymeren Ummantelung, auf. Bevorzugte Superabsorbentien sind aus der Gruppe Polyacrylate, Polymethacrylate, Stärke, Hydroxyethylcellulose, Hyaluronsäure, lineare Polysaccharide, Gelatine, Carrageen, Pektine und Mischungen davon ausgewählt.

Neben den im vorherigen Abschnitt erwähnten Additiven kann der erfindungsgemäße Faden weitere Additive, insbesondere Wirkstoffe, aufweisen. Die Wirkstoffe können dabei aus der Gruppe antimikrobielle, desinfizierende, entzündungshemmende, wachstumsfördernde und geruchsbekämpfende Wirkstoffe ausgewählt sein.

Sind quellbare Additive in der polymeren Ummantelung enthalten, kann es von Vorteil sein, wenn die polymere Ummantelung selbst eine gewisse Elastizität aufweist. Auf diese Weise kann nämlich verhindert werden, dass die Ummantelung wegen der quellenden Additive brüchig wird und Risse bekommt oder sogar Material von der polymeren Ummantelung abgesprengt wird. Bei Anwesenheit von quellbaren Additiven in der Ummantelung kann es weiterhin von Vorteil sein, wenn die polymere Ummantelung eine gewisse Hydrophilie aufweist, vorzugsweise ohne dabei selbst wasserlöslich zu sein. Dadurch kann in Körperflüssigkeiten enthaltenes Wasser zu den quellbaren Additiven in die polymere Ummantelung diffundieren und den Quellungsvorgang initiieren. Über die Hydrophilie der Ummantelung kann somit die Diffusionsgeschwindigkeit und der zeitliche Verlauf der Quellung eingestellt werden. Erfindungsgemäß ist es daher besonders bevorzugt, wenn die polymere Ummantelung hydrophil, wasserunlöslich und zumindest teilelastisch, insbesondere vollständig elastisch, ausgebildet ist.

In einer weitergehenden Ausführungsform weist die polymere Ummantelung ein hydrophiles, wasserunlösliches und vorzugsweise zumindest teilelastisches, insbesondere vollständig elastisches, Polymer auf. Bei dem Polymer kann es sich grundsätzlich um ein Homo-, Co-, Tri-, Tetrapolymer usw. handeln. Unter Copolymeren sollen im Folgenden Polymere verstanden werden, welche aus zwei oder mehreren verschiedenen Monomereinheiten zusammengesetzt sind. Insbesondere kann das Polymer als Blockco- oder Blockterpolymer oder als segmentiertes Polymer vorliegen. Des Weiteren kann das Polymer ein Elastomer, insbesondere thermoplastisches Elastomer, sein. Das Elastomer kann dabei in unvernetzter Form, insbesondere in nicht vulkanisierter Form, vorliegen. Bevorzugt ist das Polymer aus der Gruppe Polyurethane, Polyesterether, Mischungen davon und Copolymere davon, insbesondere aus der Gruppe segmentierte Polyurethane, segmentierte Polyesterether, Mischungen (Blends) davon und Copolymere davon, ausgewählt. Bei den Polyurethanen kann es sich insbesondere um lineare und vorzugsweise aliphatische Polyurethane handeln. Ein Beispiel für ein geeignetes Polyurethan ist das von B.Braun Melsungen AG unter der internen Bezeichnung Vasomer^{®} eingesetzte Polyurethan.

Die polymere Ummantelung weist in einer besonders vorteilhaften Ausführungsform eine in Körperflüssigkeiten quellbare Polymermaterix auf. Erfindungsgemäß kann es insbesondere vorgesehen sein, dass die polymere Ummantelung aus einer in Körperflüssigkeiten quellbaren Polymermatrix gebildet ist. Besonders bevorzugt weist die Polymermatrix ein hydrophiles, wasserunlösliches und zumindest teilelastisches, insbesondere vollständig elastisches, Polymer und in Körperflüssigkeiten quellbare Additive, vorzugsweise Superabsorbentien, auf. In der Regel wird die Polymermatrix selbst von einem hydrophilen, wasserunlöslichen und zumindest teilelastischen, insbesondere vollständig elastischen, Polymer gebildet, wobei in die Polymermatrix in Körperflüssigkeiten quellbare Additive, vorzugsweise Superabsorbentien, eingebettet vorliegen. Zusätzlich zu den bisher beschriebenen Vorteilen ist bei dieser Ausführungsform vorteilhaft, dass durch eine Einbindung der quellbaren Additive, insbesondere Superabsorbentien, in eine Polymermatrix im Wesentlichen ein direkter Kontakt zwischen ionischen Gruppen der quellbaren Additive, insbesondere Superabsorbentien, und Körperflüssigkeiten, insbesondere Blut, vermieden werden kann. Bezüglich weiterer Merkmale und Einzelheiten wird auf die bisher gemachten Ausführungen verwiesen.

Zur Erhöhung der Hydrophilie der polymeren Ummantelung kann es weiterhin vorgesehen sein, dass die polymere Ummantelung ein Polymerblend (Polymermischung) aufweist. Das Polymerblend weist bevorzugt ein hydrophiles, wasserunlösliches und vorzugsweise zumindest teilelastisches, insbesondere vollständig elastisches, Polymer sowie ein hydrophiles und vorzugsweise wasserlösliches Polymer auf. Bevorzugt ist das hydrophile und vorzugsweise wasserlösliche Polymer aus der Gruppe Polyethylenglykol, Polypropylenoxid, Polytetramethylenoxid, Polyvinylpyrrolidon, Polyvinylalkohol, Mischungen davon und Copolymere davon ausgewählt. Zur weiteren Erhöhung der Hydrophilie der polymeren Ummantelung können die bisher beschriebenen Polymere auch mit Monomeren, die ionische Gruppen, beispielsweise Carboxylat- und/oder Sulfonatgruppen, aufweisen, gepfropft oder copolymerisiert werden.

In einer weiteren Ausführungsform liegt die polymere Ummantelung als Hydrogel vor oder aber ist bei Kontakt mit Körperflüssigkeiten in ein Hydrogel überführbar. Ein Hydrogel besitzt im Gegensatz zu Superabsorbern in der Regel im Wesentlichen keine ladungstragenden Gruppen und kann beispielsweise durch eine physikalische oder chemische Vernetzung von wasserlöslichen oder zumindest stark hydrophilen Polymeren gebildet werden. Geeignete Vernetzungsmethoden werden im Folgenden noch eingehender beschrieben.

Erfindungsgemäß ist es daher weiterhin bevorzugt, wenn die polymere Ummantelung ein vernetzbares, insbesondere chemisch und/oder physikalisch vernetzbares, Polymer aufweist, wobei es sich bei dem vernetzbaren Polymer in der Regel um ein wasserlösliches oder zumindest stark hydrophiles Polymer handelt. Ein wasserlösliches Polymer wird in der Regel infolge einer Vernetzung wasserunlöslich. Beispiele für geeignete Polymere können aus der Gruppe Polyvinylalkohol, Polyvinylpyrrolidon, Mischungen davon und Copolymere davon ausgewählt werden. Mischungen können zudem auch Polyethylenglykol enthalten. Erfindungsgemäß kann es weiterhin vorgesehen sein, dass die polymere Ummantelung ein bereits vernetztes, insbesondere chemisch und/oder physikalisch vernetztes, und vorzugsweise wasserunlösliches Polymer aufweist.

In einer weiteren vorteilhaften Ausführungsform ist der polymere Fadenkern in Körperflüssigkeiten nicht quellbar. Vorzugsweise ist der polymere Fadenkern aus einem in Körperflüssigkeiten nicht quellbaren Polymer gebildet. Dadurch wird gewährleistet, dass der chirurgische Faden insgesamt eine mechanische Grundfestigkeit, insbesondere in Bezug auf lineare Reißkraft, Bruchdehnung, Knotenreißkraft und Knotenbruchdehnung, behält. Bevorzugt ist der polymere Fadenkern aus einem Polymer aus der Gruppe Polyolefine, Polyester, Polyamide, Mischungen davon und Copolymere davon gebildet. Beispielsweise ist das Polymer für den polymeren Fadenkern aus der Gruppe Polyethylen, Polypropylen, Polyethylenterephthalat, Mischungen davon und Copolymere davon ausgewählt.

Die polymere Ummantelung weist in einer weitergehenden Ausführungsform einen Anteil zwischen 15 und 60 Vol.-%, insbesondere 20 und 45 Vol.-%, auf, bezogen auf das Gesamtvolumen des Fadens. Der polymere Fadenkern kann dementsprechend einen Anteil zwischen 85 und 40 Vol.-%, insbesondere 80 und 55 Vol.-%, bezogen auf das Gesamtvolumen des Fadens, aufweisen.

Der erfindungsgemäße Faden weist vorzugsweise einen kreisförmigen Querschnitt auf. Erfindungsgemäß sind auch andere Querschnittsformen denkbar. Beispielsweise kann der Faden einen ovalen, dreieckigen bzw. trilobalen, quadratischen, trapezoidalen, rhomboiden, pentagonalen bzw. fünfeckigen, hexagonalen bzw. sechseckigen, stern- oder kreuzförmigen Querschnitt aufweisen. Derartige Querschnittsformen lassen sich beispielsweise ohne Weiteres mit Hilfe entsprechender Extrusionsstempel, welche kundenspezifisch mit jeder gewünschten Querschnittsform hergestellt werden können, realisieren.

Die polymere Ummantelung weist in einer weiteren Ausführungsform eine einheitliche bzw. gleichmäßige Schichtdicke auf. Besonders bevorzugt weist die polymere Ummantelung einen Radiusanteil zwischen 8 und 37 %, insbesondere 11 und 26 %, auf, bezogen auf den Gesamtradius eines im Querschnitt kreisförmigen Fadens. Der polymere Fadenkern kann erfindungsgemäß einen Radiusanteil zwischen 92 und 63 %, insbesondere 89 und 74 %, aufweisen, bezogen auf den Gesamtradius eines im Querschnitt kreisförmigen Fadens.

Weiterhin kann der polymere Fadenkern einen Durchmesser zwischen 0,07 und 1 mm, insbesondere 0,1 und 0,7 mm, aufweisen. Der Faden selbst weist vorzugsweise einen Durchmesser zwischen 0,1 und 1,5 mm, insbesondere 0,15 und 1,0 mm, auf.

Der chirurgische Faden kann grundsätzlich als Monofilament oder Multifilament vorliegen. Bevorzugt liegt der chirurgische Faden als Monofilament vor. Der chirurgische Faden kann weiterhin als sogenanntes Pseudomonofilament vorliegen. Unter einem Pseudomonofilament soll im Sinne der vorliegenden Erfindung ein chirurgischer Faden verstanden werden, dessen polymerer Fadenkern entweder aus zwei, drei oder mehreren, insbesondere aus einer Vielzahl von in der Regel sehr feinen, Monofilamenten, oder aus einem Multifilamentgarn gebildet ist, die bzw. der gemeinsam von der polymeren Ummantelung umgeben werden bzw. wird.

Bei dem chirurgischen Faden handelt es sich vorzugsweise um ein chirurgisches Nahtmaterial.

Um das Risiko von Stichkanalblutungen und post-operativen Infektionen zusätzlich zu minimieren, kann es erfindungsgemäß außerdem vorgesehen sein, dass der Faden im Bereich eines Endes oder beider Enden einen verjüngten Durchmesser aufweist. Dadurch kann der Faden mit besonderem Vorteil mit einer chirurgischen Nadel kombiniert werden, welche an sich für kleinere Fadendurchmesser ausgelegt ist. Auf diese Weise kann eine Angleichung des Fadendurchmessers an den Nadeldurchmesser bereits in trockenem Zustand des Fadens erzielt werden. Erfindungsgemäß kann dabei ein Durchmesserverhältnis von Nadel zu Faden < 2:1, vorzugsweise von 1:1, vorgesehen sein. Beispielsweise kann der verjüngte Durchmesser im Bereich der Fadenenden dem Durchmesser des polymeren Fadenkerns entsprechen, wohingegen die übrigen Fadenbereiche vorzugsweise den ursprünglichen Fadendurchmesser (einschließlich der Dicke der polymeren Ummantelung) aufweisen. Insbesondere kann der polymere Fadenkern einen Durchmesser aufweisen, der dem Durchmesser einer Nadelbohrung entspricht, und dessen Gesamtdurchmesser (einschließlich der Dicke der polymeren Ummantelung) dem Durchmesser der Nadel entspricht. Dadurch kann der durch die Nadel geformte Stichkanal von den nicht verjüngten Fadenbereichen in vorteilhafter Weise vollständig und insbesondere abdichtend ausgefüllt werden. Ein weiterer Vorteil der in diesem Abschnitt beschriebenen Ausführungsformen liegt darin, dass der Fadendurchmesser infolge der quellbaren Eigenschaften der polymeren Ummantelung den Nadeldurchmesser und damit den Durchmesser des durch die Nadel gebildeten Stichkanals bei Kontakt mit Körperflüssigkeiten deutlich überschreitet. Dies wiederum bewirkt einen besonders dichten Verschluss von Stichkanälen. Zur Verjüngung des Durchmessers kann der Faden im Bereich seiner Enden beispielsweise abgeschält werden. Erfindungsgemäß kann es hierbei vorgesehen sein, dass im Bereich der Fadenenden die polymere Ummantelung vollständig abgeschält wird. Zur Abschälung der Fadenenden können thermische Methoden, beispielsweise Lasermethoden, eingesetzt werden. Solche Abschälungstechniken bieten sich insbesondere dann an, wenn die polymere Ummantelung bei niedrigeren Temperaturen schmilzt als der polymere Fadenkern. Eine weitere geeignete Abschältechnik für einen Faden, dessen Ummantelung ein vernetzbares und wasserlösliches Polymermaterial aufweist, besteht darin, die Fadenenden, zum Beispiel über die Länge einer Nadelbohrung, in Wasser oder in einer wässrigen Lösung einzutauchen und die übrigen Fadenbereiche anschließend einer Vernetzung zu unterwerfen. Der Übergang vom ursprünglichen Durchmesser des Fadens zu einem verjüngten Durchmesser im Bereich der Fadenenden kann abrupt oder kontinuierlich, insbesondere in Form eines Gradienten, ausgebildet sein. Zur Ausbildung eines graduellen Überganges eignet sich insbesondere die Extrusionstechnik. So kann beispielsweise die Abzugsgeschwindigkeit beim Extrudieren eines Fadens variiert, insbesondere periodisch variiert, werden. Dies kann beispielsweise dadurch geschehen, dass die Umdrehungsgeschwindigkeit der für den Abzug des Fadens verantwortlichen Galette moduliert wird. Alternativ können zusätzliche Galetten zwischen der Extrusionsdüse und der Abzugsgalette geschaltet sein.

In einer weiteren Ausführungsform ist zumindest ein Ende, insbesondere ein Ende, des Fadens mit einer chirurgischen Nadel verbunden. Erfindungsgemäß ist aber auch möglich, dass beide Fadenenden mit einer chirurgischen Nadel verbunden sind. Zur Verbindung an eine chirurgische Nadel wird üblicherweise ein Teil des Fadens in eine hierfür vorgesehene Lochbohrung der Nadel eingeführt und die Nadel anschließend im Bereich der Lochbohrung gecrimpt bzw. gebördelt.

Erfindungsgemäß kann es weiterhin vorgesehen sein, dass der Faden in sterilisierter und insbesondere konfektionierter Form vorliegt. Zur Sterilisierung des Fadens kommen grundsätzlich alle dem Fachmann bekannten Sterilisationsverfahren, insbesondere γ-Sterilisierung, Elektronenbestrahlung, Röntgenbestrahlung, Ethylenoxidbegasung und/oder Plasmasterilisierung, in Betracht. Zur Konfektionierung des Fadens wird dieser in der Regel auf eine bestimmte Länge abgelängt und anschließend in geeigneten Verpackungen, beispielsweise Blisterverpackungen, steril verpackt.

Ein weiterer Aspekt der vorliegenden Erfindung betrifft ein chirurgisches Kit bzw. Set, umfassend den erfindungsgemäßen Faden sowie zumindest ein chirurgisches Einführinstrument, vorzugsweise eine chirurgische Nadel. Erfindungsgemäß kann es dabei durchaus vorgesehen sein, dass das Kit bzw. Set zwei chirurgische Nadeln aufweist, welche jeweils zur Befestigung mit einem Fadenende vorgesehen sind. Bezüglich weiterer Merkmale und Einzelheiten zu dem Kit bzw. Set wird vollumfänglich auf die bisherige Beschreibung verwiesen.

Die vorliegende Erfindung betrifft weiterhin auch ein Verfahren zur Herstellung des chirurgischen Fadens, wobei eine polymere Fadenkernkomponente und eine in Körperflüssigkeiten quellbare, polymere Ummantelungskomponente unter Ausbildung von Fäden mit einem polymeren Fadenkern und einer den Fadenkern umgebenden, in Körperflüssigkeiten quellbaren, polymeren Ummantelung coextrudiert werden.

Bei einem alternativen Herstellungsverfahren für den chirurgischen Faden wird eine fadenförmige, polymere Fadenkernkomponente, vorzugsweise durch Ummantelungsextrusion, mit einer in Körperflüssigkeiten quellbaren, polymeren Ummantelungskomponente unter Ausbildung von Fäden mit einem polymeren Fadenkern und einer den Fadenkern umgebenden, in Körperflüssigkeiten quellbaren, polymeren Ummantelung beschichtet. Abgesehen von einer Ummantelungsextrusion kann die fadenförmige, polymere Fadenkomponente auch durch Eintauch- und/oder Sprühtechniken mit der in Körperflüssigkeiten quellbaren, polymeren Ummantelungskomponente beschichtet werden. Beispielsweise kann die Fadenkernkomponente durch Eintauchen in eine wässrige Lösung der Ummantelungskomponente mit dieser beschichtet werden. Alternativ oder in Kombination dazu kann die Fadenkernkomponente für die Beschichtung auch durch eine wässrige Lösung der Ummantelungskomponente gezogen werden. Alternativ oder in Kombination dazu kann es weiterhin vorgesehen sein, die Fadenkernkomponente mit einer wässrigen Lösung der Ummantelungskomponente zu besprühen.

Für eine Coextrusion werden Fadenkernpolymer und Ummantelungspolymer in der Regel in hierfür geeigneten Extrudern, beispielsweise Doppelschneckenextrudern, welche gleich- oder gegenläufig ausgebildet sein können, aufgeschmolzen und unter erhöhtem Druck und erhöhter Temperatur möglichst gleichmäßig aus einer formgebenden Zweistoffdüse herausgepresst. Die Coextrusion kann als sogenannte Bikomponenten-Extrusion durchgeführt werden. Bei der Bikomponenten-Extrusion bestehen die Schmelzen für den Fadenkern und die polymere Ummantelung jeweils aus einem unterschiedlichen Polymer. Die Bikomponenten-Extrusion stellt ein besonders kostengünstiges Herstellungsverfahren dar. Insbesondere lassen sich mit der Bikomponenten-Extrusion Fäden mit einer besonders guten Kern-Mantel-Haftung herstellen. Eine Ummantelungsextrusion hat den Vorteil, dass als Fadenkernkomponente auch Mono- oder Multifilamente, insbesondere Multifilamentgarne, verwendet werden können. Werden Multifilamente für die Fadenkernkomponente gewählt, so lassen sich auf diese Weise Pseudomonofilamente herstellen.

In einer bevorzugten Ausführungsform wird für die Ummantelungskomponente ein hydrophiles, wasserunlösliches und vorzugsweise zumindest teilelastisches, insbesondere vollständig elastisches, Polymer verwendet. Gegebenenfalls können auch Polymerblends verwendet werden. Bezüglich weiterer Merkmale und Einzelheiten, insbesondere im Hinblick auf geeignete Polymere bzw. Polymerblends, wird vollständig auf die bisherige Beschreibung Bezug genommen.

In einer weiteren Ausführungsform werden zur Bereitstellung der Ummantelungskomponente in ein hydrophiles, wasserunlösliches und zumindest teilelastisches, insbesondere vollständig elastisches, Polymer quellbare Additive, vorzugsweise Superabsorbentien bzw. Superabsorber, eingearbeitet. Besonders bevorzugt werden die quellbaren Additive in Form eines Masterbatches oder Compounds in das Polymer eingearbeitet. Unter einem Masterbatch bzw. Compound soll im Sinne der vorliegenden Erfindung ein Konzentrat der quellbaren Additive in einem geeigneten Polymer verstanden werden. Der Masterbatch bzw. das Compound kann beispielsweise auf Knetern oder Doppelschneckenextrudern hergestellt werden. Handelt es sich bei den quellbaren Additiven um Superabsorber, kann es vorteilhaft sein, diese vor ihrer Verwendung mechanisch zu zerkleinern, beispielsweise mit Hilfe von Kugelmühlen oder Schneidmühlen.

Bei einem weiteren alternativen Herstellungsverfahren für den chirurgischen Faden wird eine fadenförmige, polymere Fadenkernkomponente in eine wässrige Lösung einer vernetzbaren, wasserlöslichen und polymeren Ummantelungskomponente eingetaucht, durch eine wässrige Lösung einer vernetzbaren, wasserlöslichen und polymeren Ummantelungskomponente gezogen oder mit einer wässrigen Lösung einer vernetzbaren, wasserlöslichen und polymeren Ummantelungskomponenten besprüht und die Ummantelungskomponente unter Ausbildung von Fäden mit einem polymeren Fadenkern und einer den Fadenkern umgebenden, in Körperflüssigkeiten quellbaren, polymeren Ummantelung vernetzt.

Auch bei diesem Herstellungsverfahren können für die Fadenkernkomponente Mono- oder Multifilamente, insbesondere Multifilamentgarne, verwendet werden. Zur Verbesserung der Löslichkeit der Ummantelungskomponente kann die wässrige Lösung Anteile von organischen Lösungsmitteln enthalten. Beispiele für geeignete Lösungsmittel sind Methanol, Ethanol, Isopropanol, Aceton und/oder Mischungen davon. Die Ummantelungskomponente kann in der wässrigen Lösung einen Anteil zwischen 3 und 30 Gew.-%, insbesondere 7 und 25 Gew.-%, aufweisen, bezogen auf das Gesamtgewicht der wässrigen Lösung. Nach dem Eintauchen bzw. Durchzug der Fadenkernkomponente in bzw. durch die wässrige Lösung der Ummantelungskomponente, insbesondere nach der Vernetzung der Ummantelungskomponente, wird der hergestellte Faden in der Regel noch getrocknet. Die Trocknung kann unter Wärme, zum Beispiel in einem hierfür geeigneten Wärmeofen, oder im Vakuum, gegebenenfalls ebenfalls unter Wärme, erfolgen.

In einer bevorzugten Ausführungsform wird die Ummantelungskomponente einer Vernetzung, insbesondere einer chemischen und/oder physikalischen Vernetzung, unterworfen. Die Vernetzung, insbesondere eine Strahlenvernetzung, wird vorzugsweise bei erhöhten Temperaturen, insbesondere in einem Temperaturbereich zwischen 50°C und 95°C, vorgenommen, um die Beweglichkeit der Polymerketten der Ummantelung zu erhöhen. Bevorzugt wird die Vernetzung nach der Coextrusion oder Ummantelungsextrusion bzw. nach dem Eintauchen in die wässrige Lösung oder nach dem Durchzug durch die wässrige Lösung der Ummantelungskomponente vorgenommen.

In einer weitergehenden Ausführungsform wird die Vernetzung mit einem Vernetzungsmittel aus der Gruppe Carbodiimide, Divinylsulfon, N-Methylenbisacrylamid, Epichlorhydrin, bi- oder oligofunktionelle Aldehyde, polyfunktionelle Polyaldehyde, bi- oder oligofunktionelle Carbonsäuren, bi- oder oligofunktionelle Ester, polyfunktionelle Polycarbonsäuren, polyfunktionelle Polyester und Borax durchgeführt. Ein Beispiel für einen geeigneten bifunktionellen Aldehyd ist Glutaraldehyd. Eine geeignete bifunktionellle Carbonsäure, insbesondere Dicarbonsäure, ist Maleinsäure (cis-Butendisäure).

In einer weiteren geeigneten Ausführungsform wird die Vernetzung radikalisch, insbesondere unter Einwirkung von UV-Licht und vorzugsweise in Gegenwart von geeigneten Radikalstartern, durchgeführt. Beispielsweise kann Polyvinylpyrrolidon unter Einwirkung von UV-Licht mit einer Wellenlänge von ca. 360 nm und in Gegenwart eines geeigneten Initiators, beispielsweise 4,4'-Diazidostilben-2,2'-Dinatriumsulfonat, vernetzt werden. Eine alternative radikalische Vernetzungsmethode sieht Temperaturen zwischen 70 und 90 °C in Gegenwart geringer Mengen eines Peroxids, beispielsweise t-Butylperoxipivalat oder H₂O₂/CuCl₂, vor. Diese Vernetzungsmethode eignet sich insbesondere für hochmolekulare Polyvinylpyrrolidon-Typen.

Die Vernetzung wird in einer weiteren vorteilhaften Ausführungsform unter Einwirkung von ionisierender Strahlung, beispielsweise β-Strahlung, γ-Strahlung, Elektronenstrahlung oder Röntgenstrahlung, vorgenommen. Zur Vernetzung von Polyvinylalkohol oder Polyvinylpyrrolidon kann beispielsweise mit einer Strahlendosis zwischen 10 und 50 kGy (kilo Gray), bevorzugt 20 und 40 kGy (kilo Gray), gearbeitet werden. Durch eine ionisierende, insbesondere strahleninduzierte, Vernetzung kann mit besonderem Vorteil auch gleichzeitig eine Sterilisierung des Fadens erzielt werden.

Mit Hinblick auf das Herstellungsverfahren für den chirurgischen Faden, welches das Eintauchen der Fadenkernkomponente in eine wässrige Lösung der Ummantelungskomponente bzw. den Durchzug durch eine wässrige Lösung der Ummantelungskomponente vorsieht, kann die Vernetzung auch in der wässrigen Lösung vorgenommen werden. So kann während oder kurz nach der Eintauch- bzw. Durchzugsphase der Fadenkernkomponente vernetzt werden. Eine geeignete Vernetzungsmethode sieht eine pH-Wert-Änderung, gegebenenfalls unter gleichzeitiger Temperaturänderung, der wässrigen Lösung oder ein Eintauchen einer mit der Ummantelungskomponente ummantelten Fadenkernkomponente in eine alkalische Lösung vor. Beispielsweise kann eine Vernetzung in einer wässrigen Lösung, welche niedermolekulare Polyvinylpyrrolidon-Typen enthält, durch Erhöhung des pH-Wertes auf Werte > 11 und bei erhöhten Temperaturen erzielt werden.

Durch eine Vernetzung der Ummantelungskomponente, insbesondere durch die in den vorhergehenden Ausführungsformen beschriebenen Vernetzungsmethoden, können die Eigenschaften des erfindungsgemäßen Fadens gezielt beeinflusst bzw. gesteuert werden. So vermindert ein holher Vernetzzungsgrad einerseits zwar die Quellbarkeit und insbesondere das Aufnahmevermögen der polymeren Ummantelung gegenüber Körperflüssigkeiten. Andererseits trägt ein höherer Vernetzungsgrad der Ummantelung jedoch auch zu einer höheren mechanischen Stabilität des Fadens bei als ein niedriger Vernetzungsgrad. Zudem kann eine Vernetzung der Ummantelungskomponente zu einer gleichmäßigen bzw. homogenen Verteilung von gegebenenfalls in der polymeren Ummantelung vorhandenen Additiven, vorzugsweise Superabsorbentien, führen.

In der Regel werden die Fäden nach der Coextrusion verstreckt. Im Falle der Ummantelungsextrusion oder des Herstellungsverfahrens, bei welchem die Fadenkernkomponente in eine wässrige Lösung der Ummantelungskomponente eingetaucht bzw. durch eine solche Lösung durchgezogen wird, liegt die Fadenkernkomponente dagegen in der Regel schon verstreckt vor. Die Verstreckung kann dabei kontinuierlich oder diskontinuierlich erfolgen. Bei der kontinuierlichen Verstreckung werden die Fäden in der Regel über ein Rollen- oder Galettensystem, ein sogenanntes Streckwerk, geführt, wobei die Rollen bzw. Galetten unterschiedliche Umdrehungsgeschwindigkeiten aufweisen können. Gewöhnlich weist jede nachfolgende Rolle bzw. Galette eine höhere Umdrehungsgeschwindigkeit als die vorhergehenden Rolle bzw. Galette des Strecksystems auf. Bei der diskontinuierlichen Verstreckung werden die Fäden dagegen in der Regel zwischen geeigneten Halte- bzw. Fixierelementen, beispielsweise Klemmbacken, einer Spannvorrichtung eingespannt und anschließend verstreckt. Die Verstreckung kann zudem unter Wärmezufuhr und/oder im Vakuum erfolgen.

Bezüglich weiterer Merkmale und Einzelheiten zu den erfindungsgemäßen Herstellungsverfahren, insbesondere im Hinblick auf verwendbare Polymere für die Fadenkern- und Ummantelungskomponente, wird vollständig auf die bisherige Beschreibung Bezug genommen.

Schließlich betrifft die vorliegende Erfindung auch die Verwendung des chirurgischen Fadens als Nahtmaterial bzw. chirurgisches Nahtmaterial, insbesondere zur Vermeidung von Stichkanalblutungen, vorzugsweise in der kardiovaskulären Chirurgie. Bezüglich weiterer Merkmale und Einzelheiten zu dem chirurgischen Faden wird ebenfalls auf die bisherige Beschreibung verwiesen.

Weitere Merkmale der Erfindung ergeben sich aus der nachfolgenden Beschreibung von bevorzugten Ausführungsformen anhand von Beispielen in Verbindung mit Merkmalen der Unteransprüche. Hierbei können einzelne Merkmale jeweils für sich alleine oder zu mehreren in Kombination miteinander verwirklicht sein.

### Beispiel 1:

### Ummantelung eines PET-Monofilaments mit einem Compound, bestehend aus 15 Gew.-% Superabsorber und 85 Gew.-% eines aliphatischen Polyurethans (Vasomer^{®})

Ein Superabsorber mit der Bezeichnung T5066F der Firma Stockhausen wurde gesiebt, wobei lediglich eine Fraktion mit Partikelgrößen < 50 µm verwendet wurde. Zur Herstellung eines Compounds für die Ummantelung eines Monofilaments aus Polyethylenterephthalat (PET) wurde ein Doppelschneckenextruder mit zwei Dosiervorrichtungen verwendet. Die Dosierstationen wurden dabei so eingerichtet, dass pro Zeiteinheit 15 Gew.-% des Superabsorbers und 85 Gew.-% des Polyurethans gefördert wurden. Die Zonen des Extruders wurden auf Temperaturen zwischen 130 und 160 °C beheizt. Die Spinnkopftemperatur betrug ebenfalls 160 °C. Bei einer Schneckendrehzahl von 40 U/min. wurde ein nahezu homogener Compound durch eine 3,0 mm Düse ausgedrückt und der Strang mit 3 m/min. abgezogen. Nach dem Abkühlen durch Stehenlassen bei Raumtemperatur wurden die Stränge granuliert. Der durchschnittliche Granulatdurchmesser betrug 2,3 mm. Die eigentliche Ummantelungsextrusion des PET-Monofilaments mit einem Durchmesser von 0,31 mm fand auf einem Einschneckenextruder mit einer 0,175 ccm Spinnpumpe, ausgestattet mit einer Ummantelungsdüse, statt. Die Spinnkopftemperatur wurde wie bei der Herstellung des Compounds auf 160 °C eingestellt. Die Extrusionsgeschwindigkeit, also die Durchzugsgeschwindigkeit des PET-Monofilaments, betrug 30 m/min. Die Spinnpumpenumdrehung betrug 3,2 U/min. Nach Abkühlung über eine Strecke von 15 m wurde das ummantelte Monofilament aufgespult. Der Durchmesser des ummantelten Monofilaments lag bei 0,35 mm, was einem Nahtmaterial der Stärke USP 2-0 entspricht. Der nahezu weiße Mantel des Monofilaments wies dabei eine geringe Rauhigkeit auf.

### Beispiel 2:

### Ummantelung eines PET-Multifilament-Geflechts

Ein Multifilament-Geflecht aus Polyethylenterephthalat (PET) mit einem Durchmesser nach USP von 0,32 mm wurde unter den gleichen Bedingungen, wie unter Beispiel 1 beschrieben, ummantelt. Der gemessene Pseudomonofilamentdurchmesser lag ebenfalls bei 0,35 mm. Das Pseudomonofilament erwies sich als deutlich biegeschlaffer als das ummantelte Monofilament.

### Beispiel 3:

### Bikomponentenextrusion eines Monofilaments mit Polypropylenkern und einem Mantel aus einem Compound, bestehend aus 15 Gew.-% Superabsorber und 85 Gew.-% eines aliphatischen Polyurethans (Vasomer^{®})

Auf einer Bikomponentenextrusionsanlage, welche aus einem Ein- und einem Doppelschneckenextruder und jeweils einer Spinnpumpe (0,25 ccm) pro Massestrom und einem Bikomponentenspinnkopf mit Kern-Mantel-Düse (1,2 mm, = 8) bestand, wurde ein Monofilament mit einem Kern aus Polypropylen und einem Mantel aus Superabsorbern und Polyurethan hergestellt. Zur Herstellung des Mantels wurde der unter Beispiel 1 beschriebene Compound verwendet. Der Compound lief hierzu auf dem Doppelschneckenextruder. Das eingesetzte Polypropylen vom Typ Borealis HC 11 5 FB besaß einen MFI von 2,8 (230 °C/2,16 kg).

**Tabelle 1: Extrusionsparameter**

| | Einschneckenextruder | Doppelschneckenextruder |
|---|---|---|
| Temperatur Zone 1 [°C] | 200 | 130 |
| Temperatur Zone 2 [°C] | 220 | 150 |
| Temperatur Zone 3 [°C] | 230 | 150 |
| Temperatur Leitung [°C] | 230 | 160 |
| Temperatur Spinnkopf [°C] | 195 | |
| Spinnpumpe [U/min] | 21,8 | 5,5 |
| Düse-Bad-Abstand [cm] | 4 | |
| Temperatur Quenchbad [°C] | 20-22 | |
| Abzug [m/min] | 10,0 | |
| Außendurchmesser [mm] | 0,93 | |
| Kerndurchmesser [mm] | 0,82 | |

Vor Bestimmung des Durchmessers mit einem Doppelachsen-Lasermessgerät wurde das Monofilament auf einer beheizten Trommel bei 70 °C und in einem Vakuum von 0,5 mbar über Nacht getrocknet. Anschließend wurde das Kern-Mantel-Monofilament zweistufig verstreckt, wobei die zweite Stufe der Relaxation zur Zunahme der Flexibilität diente.

**Tabelle 2: Verstreckparameter**

| | |
|---|---|
| Zulauf Galette 1 [m/min] | 2 |
| Schlitzheizer 1 [°C] | 100 |
| Galette 2 [m/min] | 16 |
| Schlitzheizer 2 [°C] | 115 |
| Galette 3 [m/min] | 14 |
| Gesamtverstreckverhältnis | 7 |
| Außendurchmesser Monofilament [mm] | 0,35 |
| Innendurchmesser Monofilament [mm] | 0,31 |

### Beispiel 4:

### Extrusion eines Monofilaments mit einem Kern aus Polypropylen und einem Mantel, bestehend aus einem PVA-PVP-Blend (70 Gew.-%/30 Gew.-%)

Der Doppelschneckenextruder der Bikomponentenmonofilanlage aus Beispiel 3 wurde mit zwei Dosierstationen versehen, um dem Extruder Polyvinylalkohol (PVA) und Polyvinylpyrrolidon (PVP) im Verhältnis von 70/30 (Gew.-%/Gew.-%) zuzuführen. Ein mechanisches Mischen und direktes Füttern des Extruders war wegen der unterschiedlichen Korngrößen (PVA als Granulat; PVP als feines Pulver) nicht möglich. Ansonsten entsprach der Versuchsaufbau dem unter Beispiel 3 beschriebenen Aufbau. Mit dem PVA-Typ Mowiflex TC232 der Firma Kuraray und dem PVP-Typ Luvitec VA64 der Firma BASF wurden zwei modifizierte Polymertypen gewählt, die sich besonders gut für eine thermoplastische Verarbeitung eignen.

**Tabelle 3: Extrusionsparameter**

| | Einschneckenextruder | Doppelschneckenextruder |
|---|---|---|
| Temperatur Zone 1 [°C] | 200 | 180 |
| Temperatur Zone 2 [°C] | 220 | 180 |
| Temperatur Zone 3 [°C] | 230 | 180 |
| Temperatur Leitung [°C] | 230 | 180 |
| Temperatur Spinnkopf [°C] | 195 | |
| Spinnpumpe [U/min] | 21,8 | 5,5 |
| Düse-Bad-Abstand [cm] | 4 | |
| Temperatur Quenchbad [°C] | 20-22 | |
| Abzug [m/min] | 10,0 | |
| Außendurchmesser [mm] | 0,94 | |
| Kerndurchmesser [mm] | 0,82 | |

Vor der Bestimmung des Durchmessers mit einem Doppelachsen-Lasermessgerät wurde das Monofilament auf einer beheizten Trommel bei 70 °C und in einem Vakuum von 0,5 mbar über Nacht getrocknet. Das Kern-Mantel-Monofilament wurde, wie unter Beispiel 3 beschrieben, nachfolgend zweistufig verstreckt, wobei die zweite Stufe der Relaxation zur Zunahme der Flexibilität diente. Der Außendurchmesser lag bei 0,36 mm, der Kerndurchmesser bei 0,31 mm.

### Beispiel 5:

### Ummantelung eines PET-Monofilaments mit einem Blend, bestehend aus PVA und PVP (70Gew.-%/30 Gew.-%)

Der im Beispiel 1 beschriebene Ummantelungsextruder wurde, wie der unter Beispiel 4 beschriebene Bikomponentenextruder, mit zwei Dosierstationen ausgerüstet, um Polyvinylalkohol (PVA) und Polyvinylpyrrolidon (PVP) im gewünschten Gewichtsverhältnis dem Extruder zuführen zu können. Die Extruder- und die Spinnkopftemperatur wurden auf 180 °C bzw. 185 °C eingestellt. Verwendet wurde ein Monofilament aus Polyethylenterephthalat (PET) mit einem Durchmesser von 0,29 mm. Die Spinnpumpendrehzahl des Extruders wurde auf 4,8 U/min. eingestellt, so dass sich, wie unter Beispiel 1 beschrieben, auch hier ein Außendurchmesser von 0,35 mm, allerdings bei einer größeren Schichtdicke des Mantels, ergab.

### Beispiel 6:

### Beschichtung eines PET-Multifilamentgarns aus Lösung mit PVA/PVP im Verhältnis von 30 Gew.-%/70 Gew.-%

Es wurden 60 g Polyvinylalkohol (PVA) des Typs Mowiol 44-88 und 140 g Polyvinylpyrrolidon (PVP) des Typs Luvitec K85 bei 80 °C unter Rühren in 1000 ml Wasser gelöst. Anschließend wurde die Lösung auf 50 °C abgekühlt. Durch diese Lösung wurde ein Multifilamentgarn aus Poly-ethylenterephthalat (PET) mit einer Fadenstärke von USP 2-0 mit einer Geschwindigkeit von 2 m/min durchgezogen. Die Anordnung der Umlenkrollen innerhalb und außerhalb des Beschichtungsbades wurden so gewählt, dass der beschichtete Faden die Badoberfläche rechtwinklig verließ. Nach Passieren eines Abstreifersystems durchlief der Faden zur Trocknung einen 2 m langen, auf 130 °C temperierten Heizkanal. Die Schichtdicke der aufgebrachten Beschichtung betrug im Mittel 15 µm.

### Beispiel 7:

### Endständiges Ablösen eines Monofilamentmantels zur Verringerung des Verhältnisses von Nadel- zu Fadendurchmesser

Das Ende des unter Beispiel 5 beschriebenen Kern-Mantel-Monofilaments (USP 2-0) wurde bis zu einer Tiefe, die der Länge einer Nadellochbohrung entsprach, in ein 70 °C heißes Wasserbad eingetaucht. Bereits nach wenigen Minuten hatte sich der Mantel des Monofilaments über die eingetauchte Länge aufgelöst. Der auf diese Weise verjüngte Bereich konnte an eine Nadel, welche dem kleineren Durchmesser USP 3-0 entsprach, adaptiert werden.

### Beispiel 8:

### Endständiges Abtragen eines Monofilamentmantels zur Verringerung des Verhältnisses von Nadel- zu Fadendurchmesser

Zum Abtragen des Mantels des unter Beispiel 5 beschriebenen Kern-Mantel-Monofilaments (USP 2-0) über eine Länge, welche der Tiefe einer Nadellochbohrung entsprach, wurde eine Vorrichtung verwendet, wie sie auch zum Abisolieren von elektrischen Kabeln zum Einsatz kommt. In einer speziellen Ausführungsform wurde die Vorrichtung elektrisch auf 180 °C erhitzt. Dadurch kam es zum Schmelzen des Monofilamentmantels, der sich auf diese Weise leichter abtragen ließ. Der verjüngte Bereich konnte an eine Nadel, welche dem kleineren Durchmesser USP 3-0 entsprach, adaptiert werden.

### Beispiel 9:

### Vernetzung eines Monofilamentmantels durch Einwirkung von β-Strahlen unter Ausbildung eines Hydrogels

Das unter Beispiel 5 beschriebene Kern-Mantel-Monofilament und das unter Beispiel 6 beschriebene Pseudomonofilament wurden einer Elektronenstrahlhärtungsanlage (ESH 150 der Firma Dürr) mit einer Transportgeschwindigkeit von 4,5 m/min zugeführt. Die Elektronenbestrahlung erfolgte mit einer Beschteunigungsspannung von 180 kV. Die Dosisleistung wurde durch die Regulierung des Strahlstromes auf ca. 25 kGy eingestellt. In einem weiteren Versuch wurden das Kern-Mantel-Monofilament aus Beispiel 5 und das Pseudomonofilament aus Beispiel 6 vor dem Einlaufen in die Elektronenstrahlhärtungsanlage angefeuchtet. Nach der Vernetzung wurden 50 cm lange Stücke über 3 Stunden bei 70 °C in destilliertem Wasser gelagert, um unvernetzte Bestandteile herauszulösen. In allen Fällen konnte eine deutliche Quellung des Mantels beobachtet werden. Anschließend wurden die Fadenstücke bei 80 °C während 24 Stunden im Hochvakuum getrocknet und anschließend ausgewogen. Die nicht angefeuchteten Materialien zeigten dabei im Mittel eine um 4 % geringere Masse als die vor der Vernetzung angefeuchteten Materialien, was zeigt, dass die Vernetzung im feuchten Zustand mit noch größerer Effizienz verläuft.

## Patentansprüche

1. Chirurgischer Faden, insbesondere zur Vermeidung von Stichkanalblutungen, mit einem polymeren Fadenkern und einer den polymeren Fadenkern umgebenden polymeren Ummantelung, wobei die polymere Ummantelung in Körperflüssigkeiten quellbar ist, **dadurch gekennzeichnet, dass** sich der polymere Fadenkern und die polymere Ummantelung entlang einer gemeinsamen Grenzfläche berühren, ohne dass der Fadenkern und die polymere Ummantelung kovalent miteinander verbunden sind.

2. Chirurgischer Faden nach Anspruch 1, **dadurch gekennzeichnet, dass** er als Extrusionsfaden, insbesondere als Coextrusionsfaden, vorzugsweise als Bikomponentenfaden, oder Ummantelungsextrusionsfaden, vorliegt.

3. Chirurgischer Faden nach Anspruch 1 oder 2, **dadurch gekennzeichnet, dass** die polymere Ummantelung in Körperflüssigkeiten quellbare Additive, vorzusgweise Superabsorbentien, aufweist.

4. Chirurgischer Faden nach einem der vorhergehenden Ansprüche, **dadurch gekennzeichnet, dass** die polymere Ummantelung einen Anteil an in Körperflüssigkeiten quellbaren Additiven, insbesondere Superabsorbentien, zwischen 2 und 20 Gew.-%, insbesondere 3 und 8 Gew.-%, aufweist, bezogen auf das Gesamtgewicht der polymeren Ummantelung.

5. Chirurgischer Faden nach einem der vorhergehenden Ansprüche, **dadurch gekennzeichnet, dass** die polymere Ummantelung ein hydrophiles, wasserunlösliches und vorzugsweise zumindest teilelastisches Polymer aufweist, wobei das Polymer vorzugsweise ausgewählt ist aus der Gruppe Polyurethane, Polyesterether, Mischungen davon und Copolymere davon, insbesondere aus der Gruppe segmentierte Polyurethane, segmentierte Polyesterether, Mischungen davon und Copolymere davon.

6. Chirurgischer Faden nach einem der vorhergehenden Ansprüche, **dadurch gekennzeichnet, dass** die polymere Ummantelung eine in Körperflüssigkeiten quellbare Polymermatrix aufweist, insbesondere aus einer in Körperflüsssigkeiten quellbaren Polymermatrix gebildet ist.

7. Chirurgischer Faden nach Anspruch 6, **dadurch gekennzeichnet, dass** die Polymermatrix ein hydrophiles, wasserunlösliches und zumindest teilelastisches Polymer und in Körperflüssigkeiten quellbare Additive, vorzugsweise Superabsorbentien, aufweist.

8. Chirurgischer Faden nach einem der vorhergehenden Ansprüche, **dadurch gekennzeichnet, dass** die polymere Ummantelung ein Polymerblend, insbesondere umfassend ein hydrophiles, wasserunlösliches und zumindest teilelastisches Polymer sowie ein hydrophiles und vorzugsweise wasserlösliches Polymer, aufweist.

9. Chirurgischer Faden nach Anspruch 8, **dadurch gekennzeichnet, dass** das hydrophile und vorzugsweise wasserlösliche Polymer aus der Gruppe Polyethylenglykol, Polypropylenoxid, Polytetramethylenoxid, Polyvinylpyrrolidon, Polyvinylalkohol, Mischungen davon und Copolymere davon ausgewählt ist.

10. Chirurgischer Faden nach einem der vorhergehenden Ansprüche, **dadurch gekennzeichnet, dass** die polymere Ummantelung ein vernetztes, insbesondere chemisch und/oder physikalisch vernetztes, und vorzugsweise wasserunlösliches Polymer aufweist.

11. Chirurgischer Faden nach Anspruch 10, **dadurch gekennzeichnet, dass** das Polymer aus der Gruppe Polyvinylalkohol, Polyvinylpyrrolidon, Mischungen davon und Copolymere davon ausgewählt ist.

12. Chirurgischer Faden nach einem der vorhergehenden Ansprüche, **dadurch gekennzeichnet, dass** der polymere Fadenkern in Körperflüssigkeiten nicht quellbar ist, vorzugsweise aus einem in Körperflüssigkeiten nicht quellbaren Polymer gebildet ist, wobei der polymere Fadenkern vorzugsweise aus einem Polymer aus der Gruppe Polyolefine, Polyester, Polyamide, Mischungen davon und Copolymere davon gebildet ist.

13. Chirurgisches Kit, umfassend einen chirurgischen Faden nach einem der vorhergehenden Ansprüche und zumindest eine chirurgisches Nadel.

14. Verfahren zur Herstellung eines chirurgischen Fadens, insbesondere nach einem der Ansprüche 1 bis 12, wobei eine polymere Fadenkernkomponente und eine in Körperflüssigkeiten quellbare, polymere Ummantelungskomponente unter Ausbildung von Fäden mit einem polymeren Fadenkern und einer den Fadenkern umgebenden, in Körperflüssigkeiten quellbaren, polymeren Ummantelung coextrudiert werden.

15. Verfahren zur Herstellung eines chirurgischen Fadens, insbesondere nach einem der Ansprüche 1 bis 12, wobei eine fadenförmige, polymere Fadenkernkomponente, vorzugsweise durch Ummantelungsextrusion, mit einer in Körperflüssigkeiten quellbaren, polymeren Ummantelungskomponente unter Ausbildung von Fäden mit einem polymeren Fadenkern und einer den Fadenkern umgebenden, in Körperflüssigkeiten quellbaren, polymeren Ummantelung beschichtet wird.

16. Verfahren zur Herstellung eines chirurgischen Fadens, insbesondere nach einem der Ansprüche 1 bis 12, wobei eine fadenförmige, polymere Fadenkernkomponente in eine wässrige Lösung einer vernetzbaren, wasserlöslichen und polymeren Ummantelungskomponente eingetaucht, durch eine wässrige Lösung einer vernetzbaren, wasserlöslichen und polymeren Ummantelungskomponente gezogen oder mit einer Lösung einer vernetzbaren, wasserlöslichen und polymeren Ummantelungskomponente besprüht wird, und die Ummantelungskomponente unter Ausbildung von Fäden mit einem polymeren Fadenkern und einer den Fadenkern umgebenden, in Körperflüssigkeiten quellbaren, polymeren Ummantelung vernetzt wird.

## Claims

1. Surgical thread, particularly for avoiding puncture channel bleeding, having a polymeric thread core and a polymeric sheath surrounding the polymeric thread core, wherein the polymeric sheath is swellable in bodily fluids, **characterized in that** the polymeric thread core and the polymeric sheath touch along a common interface without the thread core and the polymeric sheath being attached to each other by a covalent bond.

2. Surgical thread according to Claim 1, **characterized in that** it is present as extrusion thread, particularly coextrusion thread, preferably as bicomponent thread, or sheath extrusion thread.

3. Surgical thread according to Claim 1 or 2, **characterized in that** the polymeric sheath includes additives swellable in bodily fluids, preferably superabsorbents.

4. Surgical thread according to any preceding claim, **characterized in that** the polymeric sheath includes additives swellable in bodily fluids, particularly superabsorbents, in a proportion between 2% and 20% by weight and particularly 3% and 8% by weight, based on the overall weight of the polymeric sheath.

5. Surgical thread according to any preceding claim, **characterized in that** the polymeric sheath includes a hydrophilic, water-insoluble and preferably at least partly elastic polymer, wherein the polymer is preferably selected from the group consisting of polyurethanes, polyester-ethers, mixtures thereof and copolymers thereof, particularly from the group consisting of segmented polyurethanes, segmented polyester-ethers, mixtures thereof and copolymers thereof.

6. Surgical thread according to any preceding claim, **characterized in that** the polymeric sheath includes a polymer matrix swellable in bodily fluids and more particularly is formed of a polymer matrix swellable in bodily fluids.

7. Surgical thread according to Claim 6, **characterized in that** the polymer matrix includes a hydrophilic, water-insoluble and at least partly elastic polymer and additives swellable in bodily fluids, preferably superabsorbents.

8. Surgical thread according to any preceding claim, **characterized in that** the polymeric sheath includes a polymer blend, particularly comprising a hydrophilic, water-insoluble and at least partly elastic polymer and also a hydrophilic and preferably water-soluble polymer.

9. Surgical thread according to Claim 8, **characterized in that** the hydrophilic and preferably water-soluble polymer is selected from the group consisting of polyethylene glycol, polypropylene oxide, polytetramethylene oxide, polyvinylpyrrolidone, polyvinyl alcohol, mixtures thereof and copolymers thereof.

10. Surgical thread according to any preceding claim, **characterized in that** the polymeric sheath includes a crosslinked, particularly chemically and/or physically crosslinked, and preferably water-insoluble polymer.

11. Surgical thread according to Claim 10, **characterized in that** the polymer is selected from the group consisting of polyvinyl alcohol, polyvinylpyrrolidone, mixtures thereof and copolymers thereof.

12. Surgical thread according to any preceding claim, **characterized in that** the polymeric thread core is not swellable in bodily fluids and preferably is formed of a polymer not swellable in bodily fluids, wherein the polymeric thread core is preferably formed of a polymer from the group consisting of polyolefins, polyesters, polyamides, mixtures thereof and copolymers thereof.

13. Surgical kit comprising a surgical thread according to any preceding claim and at least one surgical needle.

14. Method for producing a surgical thread, particularly according to any one of Claims 1 to 12, wherein a polymeric thread core component and a polymeric sheath component swellable in bodily fluids are coextruded to form threads having a polymeric thread core and a polymeric sheath swellable in bodily fluids which surrounds the core.

15. Method for producing a surgical thread, particularly according to any one of Claims 1 to 12, wherein a thread-shaped polymeric thread core component is coated, preferably by sheath extrusion, with a polymeric sheath component swellable in bodily fluids to form threads having a polymeric thread core and a polymeric sheath swellable in bodily fluids which surrounds the core.

16. Method for producing a surgical thread, particularly according to any one of Claims 1 to 12, wherein a thread-shaped, polymeric thread core component is dipped into an aqueous solution of a crosslinkable, water-soluble and polymeric sheath component, pulled through an aqueous solution of a crosslinkable, water-soluble and polymeric sheath component or sprayed with a solution of a crosslinkable, water-soluble and polymeric sheath component, and the sheath component is crosslinked to form threads having a polymeric thread core and a polymeric sheath swellable in bodily fluids which surrounds the core.

## Revendications

1. Fil chirurgical, en particulier pour éviter des saignements des orifices de suture, présentant un noyau de fil polymère et un enrobage polymère, entourant le noyau de fil polymère, l'enrobage polymère étant gonflable dans les liquides corporels, **caractérisé en ce que** le noyau de fil polymère et l'enrobage polymère sont en contact le long d'une surface limite commune, sans que le noyau de fil et l'enrobage polymère soient reliés par covalence l'un à l'autre.

2. Fil chirurgical selon la revendication 1, **caractérisé en ce qu'**il se trouve sous forme de fil extrudé, en particulier sous forme de fil coextrudé, de préférence sous forme de fil à deux composants, ou sous forme de fil extrudé à enrobage.

3. Fil chirurgical selon la revendication 1 ou 2, **caractérisé en ce que** l'enrobage polymère présente des additifs gonflables dans les liquides corporels, de préférence des superabsorbants.

4. Fil chirurgical selon l'une quelconque des revendications précédentes, **caractérisé en ce que** l'enrobage polymère présente une proportion d'additifs gonflables dans les liquides corporels, en particulier des superabsorbants, située entre 2 et 20% en poids, en particulier entre 3 et 8% en poids, par rapport au poids total de l'enrobage polymère.

5. Fil chirurgical selon l'une quelconque des revendications précédentes, **caractérisé en ce que** l'enrobage polymère présente un polymère hydrophile, insoluble dans l'eau et de préférence au moins partiellement élastique, le polymère étant de préférence choisi dans le groupe formé par les polyuréthanes, les polyesteréthers, leurs mélanges et leurs copolymères, en particulier dans le groupe des polyuréthanes segmentés, des polyesteréthers segmentés, leurs mélanges et leurs copolymères.

6. Fil chirurgical selon l'une quelconque des revendications précédentes, **caractérisé en ce que** l'enrobage polymère présente une matrice polymère gonflable dans les liquides corporels et est en particulier formé par une matrice polymère gonflable dans des liquides corporels.

7. Fil chirurgical selon la revendication 6, **caractérisé en ce que** la matrice polymère présente un polymère hydrophile, insoluble dans l'eau et au moins partiellement élastique et des additifs gonflables dans les liquides corporels, de préférence des superabsorbants.

8. Fil chirurgical selon l'une quelconque des revendications précédentes, **caractérisé en ce que** l'enrobage polymère présente un mélange de polymères, comprenant en particulier un polymère hydrophile, insoluble dans l'eau et au moins partiellement élastique ainsi qu'un polymère hydrophile et de préférence soluble dans l'eau.

9. Fil chirurgical selon la revendication 8, **caractérisé en ce que** le polymère hydrophile et de préférence soluble dans l'eau est choisi dans le groupe formé par le polyéthylèneglycol, le poly(oxyde de propylène), le poly(oxyde de tétraméthylène), la polyvinylpyrrolidone, le poly(alcool vinylique), leurs mélanges et leurs copolymères.

10. Fil chirurgical selon l'une quelconque des revendications précédentes, **caractérisé en ce que** l'enrobage polymère présente un polymère réticulé, en particulier réticulé chimiquement et/ou physiquement, et de préférence insoluble dans l'eau.

11. Fil chirurgical selon la revendication 10, **caractérisé en ce que** le polymère est choisi dans le groupe formé par le poly(alcool vinylique), la polyvinylpyrrolidone, leurs mélanges et leurs copolymères.

12. Fil chirurgical selon l'une quelconque des revendications précédentes, **caractérisé en ce que** le noyau de fil polymère n'est pas gonflable dans les liquides corporels, de préférence formé d'un polymère non gonflable dans les liquides corporels, le noyau de fil polymère étant de préférence formé par un polymère du groupe formé par les polyoléfines, les polyesters, les polyamides, leurs mélanges et leurs copolymères.

13. Kit chirurgical, comprenant un fil chirurgical selon l'une quelconque des revendications précédentes et au moins une aiguille chirurgicale.

14. Procédé pour la fabrication d'un fil chirurgical, en particulier selon l'une quelconque des revendications 1 à 12, un composant de noyau de fil polymère et un composant d'enrobage polymère gonflable dans les liquides corporels étant coextrudés en formant des fils présentant un noyau de fil polymère et un enrobage polymère entourant le noyau de fil, gonflable dans les liquides corporels.

15. Procédé pour la fabrication d'un fil chirurgical, en particulier selon l'une quelconque des revendications 1 à 12, un composant de noyau de fil polymère en forme de fil étant revêtu de préférence par extrusion d'enrobage par un composant d'enrobage polymère gonflable dans les liquides corporels en formant des fils présentant un noyau de fil polymère et un enrobage polymère, entourant le noyau de fil, gonflable dans les liquides corporels.

16. Procédé pour la fabrication d'un fil chirurgical, en particulier selon l'une quelconque des revendications 1 à 12, un composant de noyau de fil polymère en forme de fil étant immergé dans une solution aqueuse d'un composant d'enrobage réticulable, soluble dans l'eau et polymère, tiré à travers une solution aqueuse d'un composant d'enrobage réticulable dans l'eau, soluble dans l'eau et polymère ou aspergé par une solution d'un composant d'enrobage réticulable, soluble dans l'eau et polymère et le composant d'enrobage étant réticulé en formant des fils présentant un noyau de fil polymère et un enrobage polymère entourant le noyau de fil, gonflable dans les liquides corporels.
